Europäisches Patentamt

European Patent Office

Office européen des brevets

(11) Publication number: 0 200 668 A2

# EUROPEAN PATENT APPLICATION

(21) Application number: 86630068.4

(22) Date of filing: 18.04.86

(51) Int. Cl.⁴: **A61B 17/22**

(30) Priority: 25.04.85 US 727397

(43) Date of publication of application:
10.12.86 Bulletin 86/45

(84) Designated Contracting States:
AT BE CH DE FR GB IT LI LU NL SE

(71) Applicant: FOGARTY, Thomas J.
770 Welch Road Suite 201
Palo Alto, CA 94304(US)

(72) Inventor: Finn III, James C.
1670 El Camino Apt. 201
Atherton California 94025(US)
Inventor: Fogarty, Thomas J.
770 Welch Road
Palo Alto California 94304(US)

(74) Representative: Schmitz, Jean-Marie et al
Office Dennemeyer S.à.r.l. 21-25 Allée
Scheffer P.O. Box 41
L-2010 Luxembourg(LU)

(54) **Apparatus and method for dislodging and removing occlusive objects from body passages.**

(57) An elongate toroidal balloon (16) is secured in concentric encircling relationship to a flexible catheter (24) to provide an instrument capable of dilating a body passage (12) and dislodging an occlusive object (10) within the passage. A localized connection (32,34) between the catheter and balloon (16) enables the balloon (16) to roll along the inside of the body passage (12) in response to the application of tension to the catheter (24). In certain embodiments balloon elements (16 or 52) serve to envelope the occlusive object (10) to shield the body passage (12) against abrasion by the object (10).

EP 0 200 668 A2

FIG. 1.

# APPARATUS AND METHOD FOR DISLODGING AND REMOVING OCCLUSIVE OBJECTS FROM BODY PASSAGES

## Background of the Invention

The present invention relates to a method and apparatus for dilating body passages and dislodging and removing occlusive objects therefrom. In its more specific aspects, the invention is concerned with such a method and apparatus wherein dislodgment and removal is achieved through means of an eversible balloon which is carried by a catheter so as to be rollable along the inside of a body passage in response to the application of tension to the catheter. Certain embodiments of the invention also provide for enveloping of the occlusive object within a balloon to shield the body passage from abrasion by the object.

Medical instruments to remove occlusive objects from body passages through means of snare-like devices are well-kown in the prior art. These may be seen, for example, in U.S. Patents 4,046,150; 3,108,594; 3,108,593; and 2,943,626. Such devices have had the common shortcoming that the body passage is not shielded from the occlusive object being removed. This shortcoming is of particular-concern when removing highly irregular and abrasive objects, such as ureteral calculi. Another deficiency of such prior art devices is that the passageway is not adequately dilated during removal of the object. This makes dislodgement and removal of the object difficult and increases the risk of injury to the body passage.

In an effort to overcome the above-mentioned shortcomings of the prior art snare-like devices, various types of suction and balloon-type extractors have been suggested. These may be seen, for example, in U.S. Patent No. 4,243,040; Federal Republic of Germany Patent 287,633 of 10/1979 and Patent No. 1,092,161 of 11/1960; U. K. Patent 887,456 of 1/1962; French Patent 1,272,412 of 8/1961; and U.S.S.R. Patent 457,471 of 3/1975. While these patents disclose various types of balloon devices into which an occlusive object may be drawn, they do not teach such a device wherein the balloon may be carried by a single catheter and rolled along the inside of the body passage to effect dislodgement of the object. These devices also have the common shortcoming that they employ rather complex structures, such as dual tubes which are slidable relative to one another, or cage supported membranes, which are necessarily rather large and cumbersome.

## Summary of the Invention

The principal elements of the apparatus comprise a flexible catheter and an elongate annular balloon disposed in generally concentric relationship to the catheter. The balloon has radially spaced inner and outer walls. A localized connection between the inner wall and the catheter permits the balloon to drape lengthwise to either side of the connection. A passage within the catheter communicates with the interior of the balloon and provides for the admission of fluid to selectively inflate or deflate the balloon.

In practicing the method, the catheter is directed through the body passage with the balloon in the deflated condition and draped to one side of the connection between the balloon and the catheter. Once positioned adjacent the object to be removed, the balloon is inflated to dilate the passage and then the catheter is retracted to roll the balloon along the inside of the vessel. The balloon may roll against the object to effect its dislodgement, or around the object to a condition wherein it fully envelopes the object.

A principal object of the invention is to provide a novel method and apparatus for dislodging occlusive objects from body passages through means of an eversible inflatable dilatation balloon which rolls along the inside of the body passage without imparting sliding friction force thereto.

Another object of the invention is to provide such an apparatus and method that is capable of encapsulating the occlusive object and shielding the passage from contact by the object during removal.

Still another object of the invention is to provide such a method and apparatus wherein the rollable balloon is carried by a single catheter and is not reliant upon a mechanism to impart relative movement to the ends of the balloon to encapsulate the object being removed.

Yet another object of the invention is to provide an apparatus and method which dilates the passage and gently dislodges the occlusive object by rolling a balloon element over the interior of the passage and into engagement with the object.

A further object of the invention is to provide such an apparatus and method which is capable of dilating the body passage to either side of the occlusive object being removed.

Still another object related to the latter object is to provide such a method and apparatus wherein balloons to either side of the occlusive object being removed are in intimate contact with the object to gently and effectively dilate the area of the passage within which the object is lodged.

Still another object of the invention is to provide such a method and apparatus having an envelope capable of encapsulating both an occlusive object and an entrapping device therefor.

A further and more general object of the invention is to provide a simplified method and apparatus employing elements of low profile which may be used in small or highly restricted body passages.

The foregoing and other objects will become more apparent when viewed in light of the following detailed description and accompanying drawings.

Brief Description of the Drawings

Fig. 1 is a perspective view of a first embodiment of the inventive instrument shown within a body passage being treated, with parts broken away and shown in section;

Fig. 2 is a cross-sectional view of the first embodiment instrument shown within a body passage, with the catheter, balloon and an entrapping device advancing towards an occlusive object;

Fig. 3 is a cross-sectional view of the first embodiment instrument within a body passage, with the entrapping device ensnaring the occlusive object and the balloon in a condition dilating the body passage;

Fig. 4 is a cross-sectional view of the first embodiment instrument within a body passage, with the catheter retracting to draw the entrapping device and the object ensnared therein into the balloon;

Fig. 5 is a cross-sectional view of the first embodiment instrument within a body passage, with the occlusive object and the ensnaring device enveloped within the balloon.

Fig. 6 is a cross-sectional view of the first embodiment instrument within a body passage, with the balloon deflated and retracting to remove the occlusive object from the body passage;

Fig. 7 is a cross-sectional elevational view of a second embodiment of the inventive instrument in a body passage, with solid lines showing the catheter and balloon advancing towards the occlusive object to be removed, and phantom lines showing the condition of the catheter and balloon after they have passed the object;

Fig. 8 is a cross-sectional view of the second embodiment instrument within a body passage, with the balloon inflated to dilate the passage in preparation for dislodgment of the occlusive object;

Fig. 9 is a cross-sectional view of the second embodiment instrument within a body passage, with the catheter retracting towards the occlusive object to effect its dislodgement;

Fig. 10 is a cross-sectional view of the second embodiment instrument within a body passage, with the balloon pushing the occlusive object to dislodge it from the body passage;

Fig. 11 is a cross-sectional elevational view illustrating a tip and balloon construction wherein the catheter has dual lumens, one for inflation of the balloon and one for providing a throughbore extending fully through the catheter;

Fig. 12 is a cross-sectional elevational view of a third embodiment of the inventive instrument within a body passage, with the catheters and balloon of that embodiment advancing towards an occlusive object to be removed from the body passage;

Fig. 13 is a cross-sectional view of the third embodiment instrument within a body passage, with two balloons in spanning relationship to the occlusive object and inflated to dilate the passage forwardly and rearwardly of the object;

Fig. 14 is a cross-sectional view of the third embodiment instrument within a body passage, with the inner catheter of that embodiment retracting towards the occlusive object;

Fig. 15 is a cross-sectional view of the third embodiment instrument within a body passage, with the front or distal balloon being withdrawn by the inner catheter to push the occlusive object into the rear or proximal balloon; and

Fig. 16 is a cross-sectional view of the third embodiment instrument within a body passage, with the balloons deflated and the catheters being retracted to remove the captured occlusive object.

Description of the Preferred Embodiments

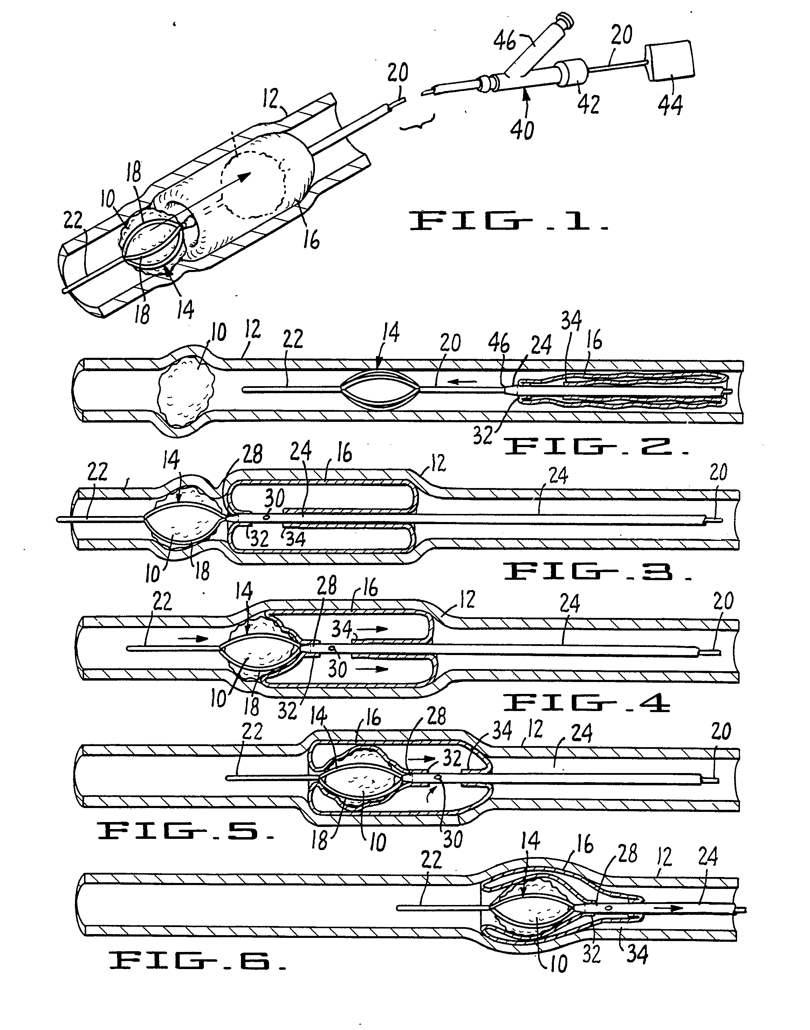

Figs 1 to 6 show a first embodiment of the invention in the process of removing an occlusive object 10 from a body passage 12, such as the urethra. In this embodiment, an entrapping snare 14 is used to draw the occlusive object into the balloon element 16 of the inventive apparatus. The snare 14 is of relatively conventional construction and includes a plurality of longitudinally extending filaments 14 adapted to pass around and entrap the occlusive object 10. The snare is carried by a wire 20. A flexible tip 22 is optionally secured to the end of the snare opposite that secured to the wire 20.

The principal elements of the first embodiment comprise a catheter 24 through which the wire 20 slidably extends and a toroidal balloon 16 fixedly secured to the catheter at a localized connection adjacent the distal end 28 of the catheter. The catheter 24 may be manufactured from any conventional material, such as polyethylene, polyvinylchoride, polyurethane, nylon, one of the fluorocarbons, or a silicone. The balloon 16 may be manufactured from either an elastomeric material, such as latex or silicone, or a nonelastomeric material, such as irradiated polyvinylchloride, polyethylene, or polyurethane.

In the preferred arrangement of the first embodiment, the balloon comprises a tubular sheath of nonelastomeric material with the ends of the sheath turned inwardly and sealingly secured to the outer periphery of the catheter 24. The connection between the balloon and catheter is "localized" in the sense that the ends of the sheath are secured to the catheter in closely spaced relationship to each other. The spacing is significantly less than the overall length of the balloon and, thus, enables the balloon to drape to either side of the connection. For example, from Fig. 2 it will be seen that the balloon is draped to the right-hand side of the connection and in Fig. 6 it will be seen that the balloon is draped to the left-hand side of the connection. It will also be seen from a comparison of Figs. 1 to 6 that the portions of the sheath making up the inner and outer walls of the balloon will vary, depending upon the draped condition of the balloon. The catheter 24 is of the dual lumen variety and includes a first lumen for slidable receipt of the wire 20 and a second lumen isolated from the first lumen for communicating fluid to and from the balloon to effect its inflation and deflation. A port 30 communicates the second lumen with the interior of the balloon. This port is disposed intermediate the sealed annular connections, designated 32 and 34, between the ends of the sheath making up the balloon and the catheter. These connections are spaced apart considerably less than the total length of the balloon and provide the "localized connection."

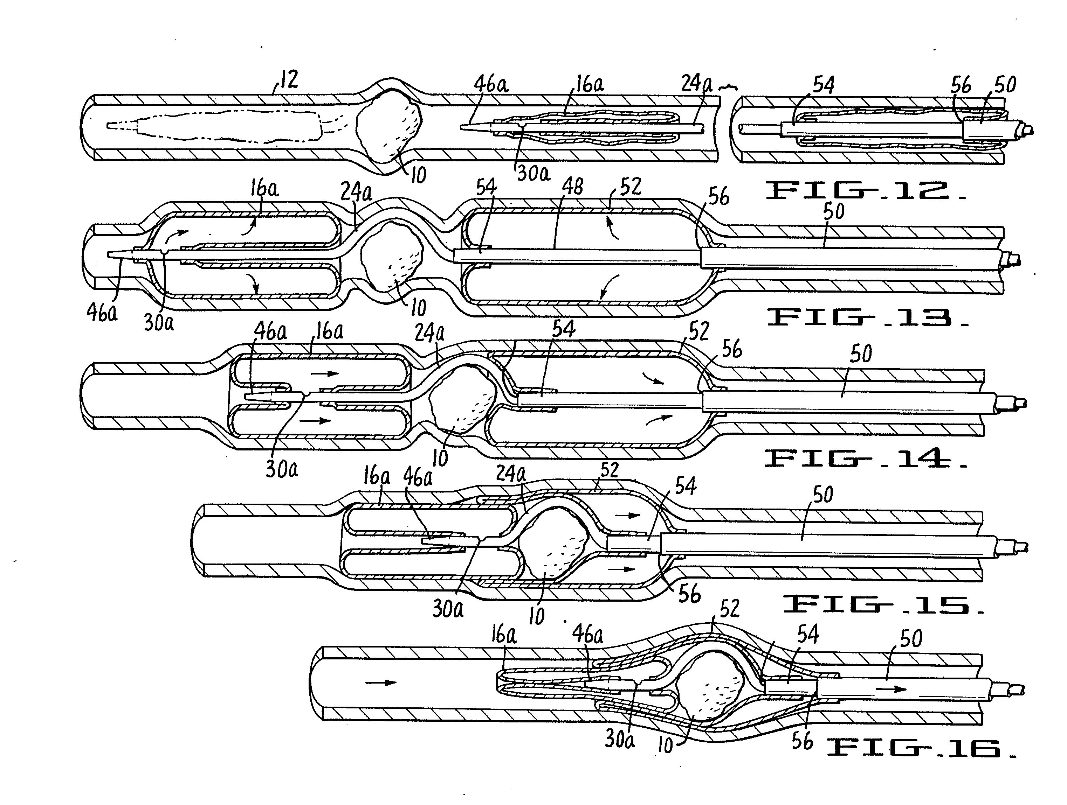

An example of a dual lumen catheter construction of the type which may be used in the first embodiment may be seen in Fig. 11. This figure shows a modified catheter and balloon construction similar to that of the first embodiment, with the exception that the outer sealed connection between the balloon element and the catheter extends outwardly, rather than inwardly of the balloon. Elements of the Fig. 11 construction corresponding to those of the first embodiment are designated by like numerals, followed by the letter "a", as follows: balloon element 16a; catheter 24a; port 30a; outer sealed annular connection 32a; and inner sealed annular connection 34a. The first and second lumens within the catheter 24a are designated by the numerals 36 and 38, respectively. The end of the lumen 36 may be enlarged, as shown at 39.

While the preferred arrangement of the first embodiment has a dual lumen catheter so that one passage of the catheter may slidably accommodate the wire 20, it is anticipated that the snare may be fixedly secured to the end of the catheter and that the wire 20 may thus be omitted. In such an embodiment, a single lumen catheter could be used, with the single lumen disposed in sealed communication with the interior of the balloon element 16 to facilitate its inflation and deflation.

As shown in Fig. 1, the proximal end of the catheter 24 is attached to a conventional Y-fitting hub 40. The proximal portion of the wire 20 passes through the hub and an O-ring seal (not illustrated) within the proximal end 42 of the fitting. A handle or knob 44 is secured to the proximal end of the wire. The hub 40 includes a side arm port 46 connected in fluid communication with the second lumen of the catheter 24. A syringe (not illustrated) is connected to the port 46 to selectively charge fluid into the balloon 16, or withdraw fluid therefrom. Thus, the hub 40 provides for sealed translation of the wire 20 relative to the catheter 24 and select inflation and deflation of the balloon.

In operation, the catheter is inserted into the body passage with the balloon in the deflated condition, as shown in Fig. 2. The snare carrying wire 14 may be fed into the body passage prior to, simultaneously with, or following insertion of the catheter. As the snare 14 approaches the occlusive object 10, the tip 22 deflects around the object and, ultimately, the snare entraps the object, as shown in Fig. 3.

With the occlusive object entrapped within the snare 14, the catheter is advanced over the wire 20 until the distal tip on the catheter, designated 46, assumes juxtaposition to the snare. When so conditioned, the next step is to inflate the balloon 16, as shown in Fig. 3, thus dilating the passage 12. Thereafter, the wire 20 and catheter 24 are simultaneously retracted, as shown in Fig. 4, to draw the snare and the object entrapped therein into the balloon. Simultaneously with the latter operation, fluid is gradually exhausted from the balloon to enable the snare and entrapped object to be fully enveloped within the balloon, as shown in Fig. 5. Once the snare and object are fully enveloped within the balloon, further fluid is withdrawn from the balloon to contract it and reduce its profile, as shown in Fig. 6. Thereafter, the wire 20 and catheter 24 are simultaneously retracted from the body passage to withdraw the occlusive object. During the course of such removal, the snare and entrapped object are fully enveloped within the balloon and thus the passage is shielded against abrasion by the object. The latter characteristic is particularly advantageous, since the object may have a highly jagged and abrasive surface. For example, it may take the form of a calculus within a urethra.

It should be appreciated that during the course of drawing the snare and occlusive object into the balloon, the outer surface of the balloon rolls inwardly around the snare and object without imparting sliding frictional force to the body passage. In other words, the outer surface of the balloon does not slide relative to the passage as the snare and object are drawn thereinto. The balloon is also maintained in a sufficiently inflated condition during this operation as to maintain the body passage in a dilated condition, as shown in Figs. 4 and 5. Once the snare and object are fully enveloped within the balloon and the balloon has deflated, as shown in Fig. 6, the balloon presents a smooth outer profile of reduced cross-section which may be drawn through the passage with relative ease. If constrictions are encountered during the course of so withdrawing the balloon from the passage, the balloon may be reinflated to generally the condition shown in Fig. 5 to further dilate the passage. Such further dilation may be repeated, as desired.

Figs. 7 to 10 show the sequential operation of a second embodiment of the invention in the process of dislodging an occlusive object from a body passage. This embodiment employs the aforedescribed balloon and catheter of Fig. 11. As shown in the sequence, it does not employ a snare to capture the object. Although not illustrated, it should be appreciated that the proximal end of the catheter of this embodiment, designated 24a, would be connected to a suitable syringe to provide for the selective inflation and deflation of the balloon element 16a. If there is no desire to use the first lumen 36, the syringe might take the form of a straight plunger syringe connected directly to the proximal end of the second lumen 38.

In practicing the dislodging technique sequentially illustrated in Figs. 7 to 10, the catheter 24a is directed into the body passage 12 with the balloon 16a in a deflated condition, as shown in Fig. 7. While the balloon is retained in the deflated condition, the catheter is advanced so that the tip 46a thereof impinges the object 10 and slides therearound. Such advancing of the catheter is continued until the balloon passes fully around the object to the position shown in phantom line in Fig. 7.

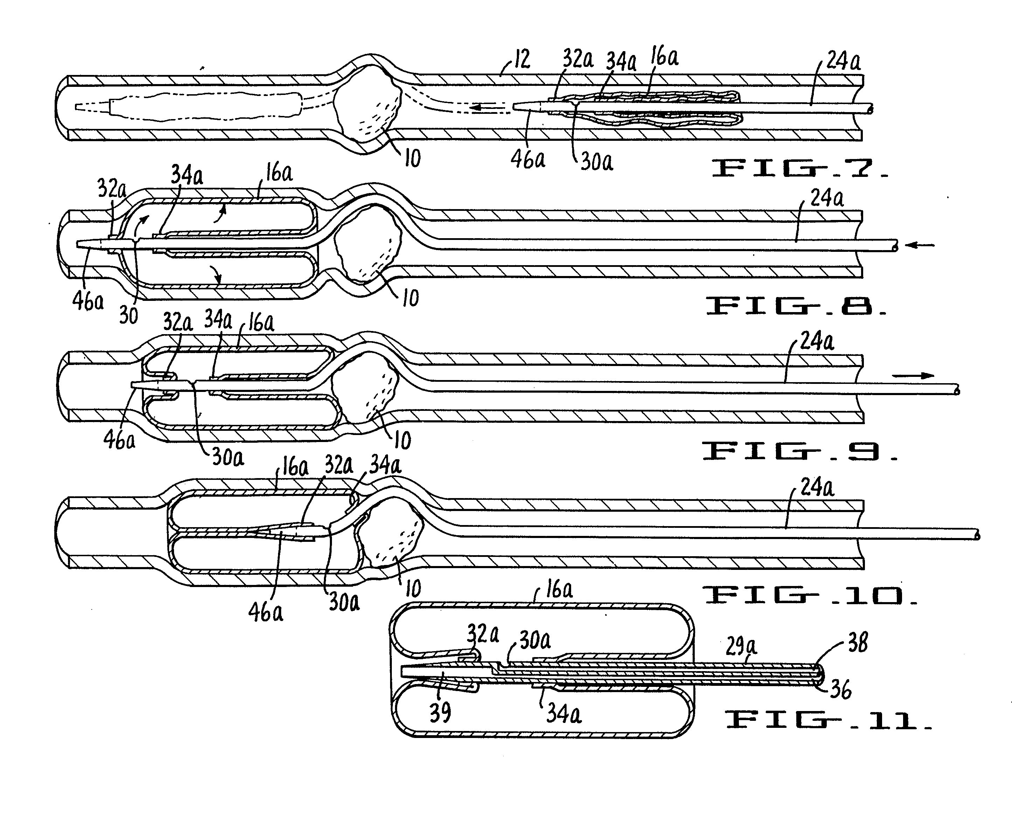

With the catheter in the latter condition, and the balloon closely adjacent the distal side of the object 10, the next step is to inflate the balloon to dilate the body passage, as shown in Fig. 8. Thereafter, the catheter 24a is gently retracted in the direction shown by the arrow line in Fig. 9 to roll the balloon inwardly upon itself and against the object 10. Such rolling of the balloon while in the inflated condition functions to dilate that body passage within which the object is captured and to gently dislodge the object. Continued withdrawal of the catheter 24a with the balloon in the inflated condition functions to fully turn the balloon in upon itself until the balloon ultimately reaches the condition shown in Fig. 10. Thereafter, continued withdrawal of the catheter 24a with the balloon in the inflated condition pushes the object through the passage.

Figs. 12 to 16 illustrate a third embodiment of the invention in the process of removing an occlusive object 10 from a body passage 12. To the extent of the distal balloon of this embodiment, it corresponds to the second embodiment, as illustrat-

ed in Figs. 7 to 10. Accordingly, the distal balloon and the catheter therefor are designated by numerals corresponding to those of the second embodiment.

The third embodiment differs from the second embodiment in that it additionally comprises: an intermediate catheter 48 slidably received upon the inner catheter 24a; an outer catheter 50 slidably received on the intermediate catheter; and a proximal annular balloon 52 concentrically received around and sealingly secured between the intermediate and outer catheter. The balloon 52 comprises a flexible sheath which may be fabricated of material corresponding to that of the aforedescribed balloon 16. One end of the sheath is turned in upon itself and sealingly secured to the distal end 54 of the intermediate catheter 48. The other end of the sheath is sealingly secured around the distal end 56 of the outer catheter 50. Although not illustrated, it should be appreciated that the proximal ends of the inner catheter 24a and the outer catheter 50 are secured in fluid communication with syringes in order that fluid may be discharged and withdrawn therethrough to selectively inflate and deflate the balloons 16a and 52. The distal end 56 of the catheter 50 provides an open annular passage around the intermediate catheter 48 through which fluid may be charged into or withdrawn from the balloon 52.

The first step in employing the third embodiment to remove an occlusive object is to direct the assembly of inner, intermediate and outer catheters through the body passage with the distal and proximal balloons in a deflated condition, as shown in Fig. 12. The catheters are thus advanced through the passage until the catheter tip 46a impinges upon the the occlusive object 10 and slides therearound to the phantom line position shown in Fig. 12. Thereafter, both the distal and proximal balloons are inflated to dilate the body passage, as shown in Fig. 13.

The next step is to gradually retract the inner catheter 24a and intermediate catheter 48, as shown in Fig. 14, while permitting fluid to expel from the proximal balloon 52 to enable the occlusive object 10 to be pushed thereinto by the distal balloon 16a. During the course of such retraction, the distal balloon rolls inwardly upon itself and against the occlusive object, and the proximal balloon is drawn in upon itself, as shown in Fig. 15. Ultimately, as shown in the latter figure, the occlusive object is fully enveloped within the proximal balloon, with the distal balloon engaged therewith. After thus conditioning the distal and proximal balloons, both balloons are deflated to the condition shown in Fig. 16 to reduce the overall profile of the balloons; and the assembled inner, intermediate and outer catheters are withdrawn from the body passage to remove the occlusive object.

It should be appreciated that the third embodiment functions to dilate the body passage to both sides of the occlusive object. This, together with the gentle dislodgment of the object into the proximal balloon by the rolling action of the distal balloon, provides an ideal system for extracting difficult to move occlusive objects. Additionally, like the other embodiments, it is anticipated that the distal end or proximal balloons may be selectively reinflated during the course of withdrawing an occlusive object from a body passage. Such selective inflation may be repeated, as desired, to selectively dilate the passage, as desired.

Conclusion

While preferred embodiments and applications of the inventions have been illustrated and described, it should be understood that the invention is not intended to be limited to the specifics of these embodiments, but rather is defined by the accompanying claims.

**Claims**

1. An instrument for removing an occlusive object from a body passage, said instrument comprising: a flexible catheter; an elongate annular balloon disposed in generally concentric relationship to the catheter, said balloon having radially spaced inner and outer walls; means securing the inner wall of the balloon to the catheter through a localized connection whereby the balloon may drape lengthwise of the catheter to either side of the connection; and means to selectively inflate and deflate the balloon.

2. An instrument according to Claim 1 wherein the catheter has proximal and distal ends and the localized connection is adjacent the distal ends, the instrument further comprising a snare secured to the distal end of the catheter for capturing an occlusive object and drawing the object into the balloon in response to retraction of the distal end of the catheter relative to the balloon.

3. An instrument according to Claim 1 wherein the catheter has a passage extending longitudinally therethrough, the instrument further comprising a wire secured to the snare and extending slidably through said passage for moving the snare relative

to the catheter and securing the snare to the distal end of the catheter.

4. An instrument according to Claim 1 wherein the balloon comprises an elongate annular sheath having the ends thereof turned inwardly to provide the inner wall of the balloon and the means securing the inner wall of the balloon to the catheter comprises fixed sealed annular connections between the ends of the sheath and the catheter, said connections being disposed so as to be closely adjacent one another and within the confines of the balloon.

5. An instrument according to Claim 1 wherein the means to selectively inflate and deflate the ballon comprises a sealed fluid passage extending longitudinally through the catheter and a port establishing fluid communication between said passage and the interior of the balloon.

6. An instrument according to Claim 5 wherein the balloon comprises an elongate annular sheath having the ends thereof turned inwardly to provide the inner wall of the balloon, the means securing the inner wall of the balloon to the catheter comprises fixed annular sealed connections between the ends of the sheath and the catheter, said connections being in closely spaced relationship to one another within the confines of the balloon, and the port extends through the catheter in a disposition between said connections.

7. An instrument for removing an occlusive object from a body passage, said instrument comprising:

an elongate flexible outer catheter having proximal and distal ends;

an elongate flexible intermediate catheter extending slidably through the outer catheter, said intermediate catheter having proximal and distal ends and extending out of the distal end of the outer catheter;

an elongate flexible inner catheter extending slidably through the intermediate catheter, said inner catheter having proximal and distal ends and extending out of the distal end of the intermediate catheter;

a first elongate annular balloon disposed in generally concentric relationship to the outer and intermediate catheters and secured between the distal ends of said catheters, said balloon being adapted to be turned in upon itself in response to retraction of the intermediate catheter relative to the outer catheter;

a second elongate annular balloon disposed in generally concentric relationship to the inner catheter, said balloon having radially spaced inner and outer walls;

means securing the inner wall of the second balloon to the inner catheter through a localized connection disposed externally of the distal end of the intermediate catheter, said means providing a localized connection whereby the balloon may be draped lengthwise of the inner catheter to either side of the connection;

means to selectively and independently inflate the first and second balloons.

8. An instrument according to Claim 7 wherein the second balloon comprises an elongate annular sheath having the ends thereof turned inwardly to provide the inner wall of said balloon and the means securing the inner wall of said balloon to the inner catheter comprise fixed sealed annular connections between the ends of the sheath and the inner catheter, said connections being disposed so as to be closely adjacent one another and within the confines of the second balloon.

9. An instrument according to Claim 7 wherein the means to selectively inflate and deflate the second balloon comprises a sealed fluid passage extending longitudinally through the inner catheter and a port establishing fluid communication between said passage and the interior of the second balloon.

10. An instrument according to Claim 9 wherein the second balloon comprises an elongate annular sheath having the ends thereof turned inwardly to provide the inner wall of the second balloon, the means securing the inner wall of the second balloon to the inner catheter comprises fixed annular sealed connections between the ends of the sheath and the inner catheter, said connections being in closely spaced relationship to one another within the confines of the second balloon, and the port extends through the inner catheter in a disposition between said connections.

11. A method for removing an occlusive object from a body passage, said method comprising: securing an elongate toroidal balloon in concentric encircling relationship to a flexible catheter through a localized connection between the balloon and catheter whereby the balloon may drape to either side of the connection; directing the catheter through the body passage with the balloon in a deflated condition and draped to one side of the connection to a position adjacent the object; inflating the balloon to dilate the passage; drawing the object into the balloon while retracting the catheter relative to the object whereby the catheter rolls round the object to a condition draped to the side of said connection opposite said one side; deflating the balloon; and withdrawing the catheter from the passage with the object within the balloon.

12. A method according to Claim 11 further comprising periodically inflating said balloon as the catheter is being withdrawn from the body passage to dilate the passage.

13. A method for dislodging an occlusive object from a body passage, said method comprising: securing an elongate toroidal balloon in a concentric encircling relationship to a flexible catheter through a localized connection between the balloon and catheter whereby the balloon may drape to either side of the connection; directing the catheter through the body passage with the balloon in deflated condition and draped to one side of the connection to move the balloon past the object to a position adjacent and beyond the object; inflating the balloon to dilate the passage; tensioning the catheter to draw the balloon from the condition draped to one side of the connection to a condition draped to the side of the connection opposite said one side to thereby roll the balloon within the passage and into engagement with the object while progressively dilating the passage.

14. A method according to Claim 13 further comprising: disposing a second elongate toroidal balloon around the catheter in closely spaced relationship to the said one side of the first mentioned balloon; directing said ballon through the body passage in a deflated condition along with the catheter to position said second balloon to the side of the object opposite that to which the first mentioned balloon is positioned; inflating said second balloon simultaneously with the inflation of said first mentioned balloon; capturing the object within said second balloon as the object is engaged by the first mentioned balloon in response to tensioning of the catheter.

15. A method according to Claim 14 wherein said second balloon is permitted to deflate to enable the object to be captured therein.

16. A method according to Claim 15 further comprising tensioning the catheter to withdraw the first mentioned and second balloons from the passage with the object captured within the second balloon.

FIG.1.

FIG.2.

FIG.3.

FIG.4

FIG.5.

FIG.6.

FIG.7.

FIG.8.

FIG.9.

FIG.10.

FIG.11.

0 200 668

FIG.12.

FIG.13.

FIG.14.

FIG.15.

FIG.16.

0 200 668